# EUROPÄISCHE PATENTSCHRIFT

(11) **EP 0 387 677 B1**
(45) Veröffentlichungstag und Bekanntmachung des Hinweises auf die Patenterteilung: **16.03.1994**
(21) Anmeldenummer: 90104323.2
(22) Anmeldetag: 07.03.1990
(51) Int. Cl.: C07D 333/24, A61K 31/38

(54) **Thienylessigsäurederivate, Verfahren zu ihrer Herstellung ihre Verwendung sowie diese enthaltende Arzneimittel und deren Herstellung**
Thienylacetic acid derivatives, process for their preparation, their application as well as pharmaceuticals containing them and their preparation
Dérivés de l'acide thiénylacétique, procédé pour leur préparation, leur application ainsi que médicaments les contenant et leur préparation

(30) Priorität: 16.03.1989 DE 3908547
(43) Veröffentlichungstag der Anmeldung: 19.09.1990
(73) Patentinhaber: CASSELLA Aktiengesellschaft, D-60386 Frankfurt (DE)
(72) Erfinder: Beyerle, Rudi, Dr., D-6000 Frankfurt am Main 60 (DE); Schönafinger, Karl, Dr., D-8755 Alzenau (DE); Schindler, Ursula, Dr., D-6238 Hofheim/Ts (DE); Jablonka, Bernd, Dr., D-6232 Bad Soden (DE); Troke, Jeffery, Dr., Newport Pagnell, Buckinghamshire (GB)
(74) Vertreter: Muley, Ralf, Dr.

(56) Entgegenhaltungen:
- EP-A- 0 072 932
- FR-A- 2 351 952
- Tetrahedron Letters vol. 26, no. 6, 1985, Great Britain Seiten 699 - 702; Martin j.O'Donnel et al.: "The Synthesis of Amino acids by reaction of an electrophilic Glycine cation equivalent with carbon nucleophiles."
- CHEMICAL ABSTRACTS, vol. 106, no. 5, 02 Februar 1987 Columbus, Ohio, USA Saletu,B et al.: "Pharmacokinetic and -dynamic studies in elderly persons with a potential antihypoxic/nootropic drug,tenilsetam,utilizing pharmaco-EEG and"psychometry" Drug.Dev.Res.1986,9(2),95-113 Seite 50; Spalte 2; ref. no. 27706D EP 90104323030

## Beschreibung

Die vorliegende Erfindung betrifft Thienylessigsäurederivate der allgemeinen Formel I, 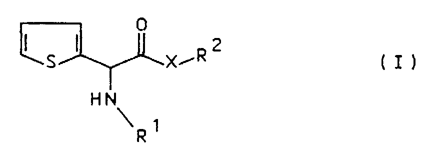
worin
X -O- oder -NH-;
R¹ Wasserstoff, die Gruppe -CH₂-CO-R³ oder die Gruppe CO-CH₂-NH₂;
R² Wasserstoff, (C₁-C₄)-Alkyl oder den Rest R¹ und
R³ (C₁-C₄)-Alkoxy, Hydroxy oder Amino bedeuten, wobei R¹ und R² nicht gleichzeitig für Wasserstoff stehen können, wenn X = O bedeutet,
sowie ihre pharmazeutisch verträglichen Salze.

Für R² stehendes (C₁-C₄)-Alkyl und der Alkylrest von für R³ stehendes (C₁-C₄)-Alkoxy sind geradkettig oder verzweigt und bedeuten Methyl, Ethyl, n-Propyl, i-Propyl, n-Butyl, i-Butyl oder tert.-Butyl. Bevorzugt sind Methyl, Ethyl und tert.-Butyl.

Pharmazeutisch verträgliche Salze sind bevorzugt Säureadditionssalze, besonders bevorzugt die Hydrochloride.

Bevorzugte Verbindungen der allgemeinen Formel I sind solche, in denen
X -O-;
R¹ die Gruppe -CH₂-CO-R³;
R² Wasserstoff oder (C₁-C₄)-Alkyl und
R³ (C₁-C₄)-Alkoxy, Hydroxy oder Amino
bedeuten.

Ebenfalls bevorzugt sind solche Verbindungen der allgemeinen Formel I, in denen
X -NH-;
R¹ die Gruppe -CH₂-CO-R³;
R² Wasserstoff und
R³ (C₁-C₄)-Alkoxy oder Hydroxy
bedeuten.

Besonders bevorzugt sind Thien-2-yl-N-aminocarbonylmethyl-aminoessigsäure und Thien-2-yl-N-hydroxycarbonylmethyl-aminoessigsäureamid-hydrochlorid.

Verbindungen der allgemeinen Formel I können beispielsweise dadurch hergestellt werden, daß eine Verbindung der allgemeinen Formel II 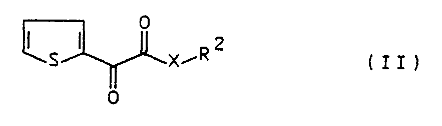
worin X und R² wie oben definiert sind, mit Hydroxylamin zum Oxim und dieses durch Reduktion zur erfindungsgemäßen Verbindung der allgemeinen Formel Ia 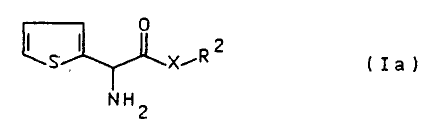
umgesetzt wird, wobei letztere gegebenenfalls
a) durch Alkylierung mit Halogenessigsäureestern der allgemeinen Formel III 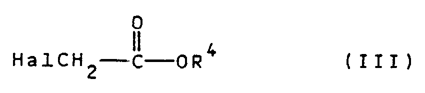 worin
   Hal Halogen, bevorzugt Brom, und
   R⁴ (C₁-C₄)-Alkyl
   bedeuten, zu weiteren erfindungsgemäßen Verbindungen der allgemeinen Formel Ib 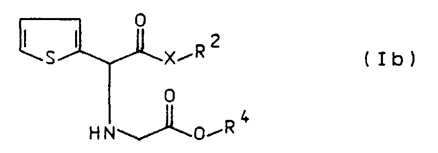 und diese durch Hydrolyse oder Ammonolyse mit Ammoniak zu weiteren erfindungsgemäßen Verbindungen der allgemeinen Formel Ic, 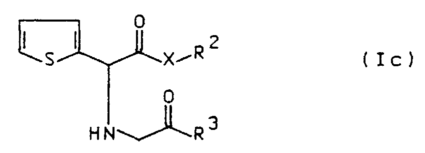 worin R³ -OH oder -NH₂ bedeutet, umgesetzt werden können, oder
b) durch Umsetzung mit dem gemischten Anhydrid der Formel IV 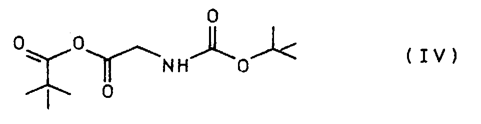 und anschließender Hydrolyse in die erfindungsgemäßen Verbindungen der Formel Id, 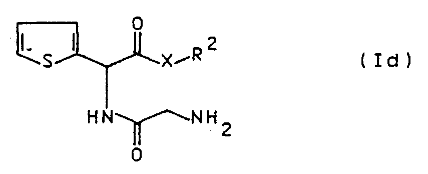 überführt werden können, wobei die erfindungsgemäßen Verbindungen der allgemeinen Formeln Ia bis Id gegebenenfalls noch in ein Salz überführt werden können.

Die Verbindungen der allgemeinen Formel II sind beispielsweise aus der käuflichen 2-Thienylglyoxylsäure durch Veresterung bzw. Amidierung bzw. aus deren Ester, wie beispielsweise aus 2-Thienylglyoxylsäureethylester durch Umesterung bzw. Aminolyse mit entsprechend substituierten Alkoholen bzw. primären Aminen zugänglich. Die Verbindung der allgemeinen Formel III sowie die genannten Alkohole und primären Amine sind bekannt und können nach den üblichen Methoden hergestellt werden.

Die Verbindung der Formel IV ist bekannt und wird vorteilhafterweise in situ aus N-tert.Butoxycarbonylglycin Trimethylessigsäurechlorid hergestellt.

Eine weitere Methode zur Herstellung erfindungsgemäßer Verbindungen der allgemeinen Formel I besteht darin, daß 2-Thienylglycin der Formel V 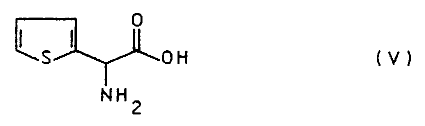
zu erfindungsgemäßen Verbindungen der allgemeinen Formel Ia verestert bzw. amidiert wird und diese gegebenenfalls, wie oben unter a) und b) beschrieben, weiter umgesetzt werden.

2-Thienylglycin ist eine käufliche Verbindung.

Bei den angegebenen Synthesemöglichkeiten können gegebenenfalls auch Verfahrensschritte in ihrer Reihenfolge vertauscht werden.

Alle zur Herstellung der Verbindungen der allgemeinen Formel I beschriebenen Verfahrensschritte, wie beispielsweise Veresterungen, Umesterungen, Ammonolysen, Amino und lysen, Amidierungen, Alkylierungen, Hydrolysen und Reduktionen, sind dem Fachmann an sich bekannt und in allen gängigen Lehrbüchern der Organischen Chemie, beispielsweise in Houben-Weyl, "Methoden der Organischen Chemie", beschrieben.

Die Reduktion der durch Umsetzung der Verbindung der Formel II mit Hydroxylamin erhaltenen Verbindung geschieht bevorzugt in Alkoholen als Lösungsmittel. Besonders bevorzugt sind Methanol und Ethanol. Die Reaktionstemperaturen liegen vorzugsweise bei 30 - 120°C. Als Katalysator werden vorzugsweise Palladium- und Platin-Katalysatoren, besonders bevorzugt Raney-Nickel, verwendet.

Alkylierungen von Verbindungen der allgemeinen Formel Ia mit Halogenessigsäureestern der allgemeinen Formel III werden vorzugsweise bei Temperaturen von 0 - 100°C ausgeführt. Als Lösungsmittel sind alle inerten Lösungsmittel bevorzugt, besonders bevorzugt sind Ether, DMF und DME. Die sich anschließende Hydrolyse zu Verbindungen der allgemeinen Formel Ic wird bevorzugt in inerten Lösungsmitteln, besonders bevorzugt in Alkoholen und Ethern, bei Temperaturen von vorzugsweise 0°C bis zum Siedepunkt des verwendeten Lösungsmittels ausgeführt.

Alkylierungen von Verbindungen der allgemeinen Formel Ia mit dem gemischten Anhydrid der Formel IV werden bevorzugt in inerten Lösungsmitteln, besonders bevorzugt in DMF oder Ether, bei Temperaturen von vorzugsweise -20 bis +60°C, besonders bevorzugt bei -20°C bis Raumtemperatur, ausgeführt. Die sich daran anschließende Hydrolyse wird vorzugsweise sauer, beispielsweise mit Chlorwasserstoff oder Trifluoressigsäure, bevorzugt bei Temperaturen von 0 bis 50°C, besonders bevorzugt bei Raumtemperatur, ausgeführt.

Sofern die erfindungsgemäßen Verbindungen der allgemeinen Formel I basische Reste enthalten, bilden sie mit anorganischen oder organischen Säuren Säureadditionssalze. Zur Bildung derartiger Säureadditionssalze sind anorganische und organische Säuren geeignet. Geeignete Säuren sind beispielsweise: Chlorwasserstoff, Bromwasserstoff, Naphthalindisulfonsäuren, insbesondere Naphthalin-1,5-disulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Nicotin-, Isonicotin-, Methansulfon-, p-Toluolsulfon-, Zitronen- oder Adipin-Säure. Pharmakologisch annehmbare Säureadditionssalze werden bevorzugt.

Die Säureadditionssalze werden wie üblich durch Vereinigung der Komponenten, zweckmäßigerweise in einem geeigneten Lösungsmittel oder Verdünnungsmittel, hergestellt. Bei der Synthese der Verbindungen der allgemeinen Formel I können zunächst die Säureadditionssalze im Zuge der Aufarbeitung anfallen. Aus den Säureadditionssalzen können die freien Verbindungen der allgemeinen Formel I gewünschtenfalls in bekannter Weise, z.B. durch Auflösen oder Suspendieren in Wasser und Alkalischstellen, z.B. mit Natronlauge, und anschließendes Isolieren, gewonnen werden.

Verbindungen der allgemeinen Formel I die eine Carboxylgruppe aufweisen, können auch in Salzform vorliegen. Bevorzugt sind die Natrium-, Kalium-und Ammoniumsalze, die durch Umsetzung der sauren Form mit entsprechenden Basen erhalten werden können. Falls die Verbindungen der allgemeinen Formel I neben einer Carboxygruppe auch eine freie Aminogruppe enthalten, können sie auch in Form innerer Salze vorliegen.

Die erfindungsgemäßen Verbindungen der allgemeinen Formel I und ihre pharmazeutisch verträglichen Salze besitzen wertvolle pharmakologische Eigenschaften. Sie sind zentralwirksam, beispielsweise zeigen sie encephalotrope und nootrope Wirkungen und dienen zur Behandlung von Krankheiten der Gehirnfunktionen wie cerebraler Insuffizienz, cerebraler Alterungsvorgänge, verminderter Gedächtnisleistung wie sie auch bei der Alzheimer Krankheit oder der Multi-Infarkt Demenz oder bei verminderter Lernleistung auftreten. Sie sind den bisher bekannten Verbindungen gleicher Wirkungsrichtung überraschenderweise erheblich überlegen. Sie zeigen eine ausgezeichnete Wirksamkeit in verschiedenenartigen Tests, wie z.B. bei der Verlängerung der Überlebenszeit unter Natriumnitrit-Hypoxie nach Gibsen und Bless (J. Neurochemistry 27, (1976)), bei der Verbesserung der stickstoffinduzierten Hypoxietoleranz, wobei Versuchstiere nach Prämedikation mit den untersuchten Präparaten mit reinem Stickstoff beatmet werden und die Verlängerung des Zeitraumes zwischen Einsatz der Beatmung und elektrischer Neutralität des Elektroencephalogramms sowie die Letalität gemessen werden.

Auch in Tests, die direkt auf die Erfassung der Lern- und Gedächtnisleistung abzielen, wie z.B. den bekannten "avoidance"-Tests, sind die erfindungsgemäßen Produkte sehr gut wirksam.

Die Prüfung in den genannten und einer Reihe weiterer Tests ergibt, daß die erfindungsgemäßen Verbindungen in niedrigen Dosen bei geringer Toxizität überraschenderweise ein besonders günstiges, bei bekannten Präparaten in dieser Form nicht vorliegendes Wirkprofil aufweisen. Dies soll anhand folgender Tabellen präzisiert werden:

### 1) Scopolamin-induzierte Amnesie bei der Maus

Die Injektion einer Scopolamin Dosis von 3 mg/kg i.p. führt zu einer Amnesie in einem Passive-avoidance Lernversuch. Die perorale Vorbehandlung mit den Verbindungen der allgemeinen Formel I kann, wie die folgende Tabelle zeigt, die experimentell induzierte Amnesie signifikant antagonisieren. Die bekannte Substanz Piracetam ist als Vergleich dazu nur marginal wirksam.

| Beispiel Nr. | Dosis mg/kg po | Scopolamin-Amnesie % Umkehr der Amnesie |
|---|---|---|
| 5 | 30 | 56 |
| 8 | 30 | 71 |
| Piracetam (Vergleich) | 30 | 18 |

### 2) Ischämie-induzierte Amnesie beim mongolischen Gerbil

Eine kurzdauernde bilaterale cerebrale Ischämie (3-5 min) führt beim Gerbil zu einer anhaltenden Lernstörung des Passive-avoidance Lernens. Werden die Tiere sofort nach dem Ende der Ischämie intraperitoneal mit den Verbindungen der allgemeinen Formel I behandelt, kann die Lernstörung antagonisiert werden. In der nachfolgenden Tabelle sind diejenigen Dosierungen angegeben, die eine halbmaximale (ca. 50%ige) Antagonisierung der Lernstörung hervorrufen. Die erfindungsgemäßen Verbindungen zeigen eine ausgeprägte Überlegenheit über Verbindungen des Standes der Technik.

| Beispiel Nr. | Dosis mg/kg ip | Ischämie-induzierte Lernstörung % Umkehr der Lernst. |
|---|---|---|
| 5 | 1,00 | 51 |
| 8 | 0,03 | 58 |
| 9 | 0,10 | 54 |
| 11 | 1,00 | 54 |
| Aniracetam (Vergleich) | 30,00 | 45 |
| Oxiracetam (Vergleich) | 100,00 | 46 |

### 3) Rezeptor-Bindungsstudien in vitro

Die Prüfung der Wirkung der erfindungsgemäßen Substanzen der allgemeinen Formel I auf die Bindungskonstanten des Glycin-Rezeptors in Rattencortex Synaptosomen läßt eine ausgeprägte spezifische Bindung erkennen. Die bekannte Substanz Piracetam ist unwirksam.

| Beispiel Nr. | Bindung an Glycin-Rezeptoren des Gehirns |
|---|---|
| 9 | Kd = 1,8 x 10⁻⁶ M |
| 11 | Kd = 0,8 x 10⁻⁶ M |
| Piracetam (Vergleich) | keine spezifische Bindung |

Die Verbindungen der allgemeinen Formel I und ihre physiologisch verträglichen Salze stellen somit eine Bereicherung der Pharmazie dar.

Die erfindungsgemäßen Verbindungen der allgemeinen Formel I und ihre pharmazeutisch verträglichen Salze können daher am Menschen als Heilmittel z.B. bei der Bekämpfung bzw. Vorbeugung von Erkrankungen, die durch eine Einschränkung der Gehirnfunktion bedingt sind und bei der Behandlung und Vorbeugung von cerebralen Alterungsvorgängen Anwendung finden.

Die Verbindungen der allgemeinen Formel I und ihre pharmazeutisch verträglichen Salze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen enthalten. Die Zubereitungen enthalten normalerweise etwa 0,5 bis 90 Gew.% der therapeutisch wirksamen Verbindung.

Die Heilmittel können oral, z.B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glycerin, Polyole, pflanzliche Öle etc.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Säureadditionssalze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanato-Glykoside; Coronardilatatoren, wie Carbocromen, Dipyridamol, Nifedipin und Perhexilin, antianginöse Verbindungen, wie Isosorbiddinitrat, Isosorbidmononitrat, Glycerolnitrat, Molsidomin und Verapamil, β-Blocker, wie Propranolol, Oxprenolol, Atenolol, Metoprolol und Penbutolol. Darüberhinaus lassen sich die Verbindungen mit anderen nootrop wirksamen Substanzen, wie z.B. Piracetam, oder ZNS-aktiven Substanzen, wie Pirlindol, Sulpirid etc. kombinieren.

Die Dosierung kann innerhalb weiter Grenzen variieren und ist in jedem einzelnen Fall den individuellen Gegebenenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Application beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z.B. 2, 3 oder 4, Teilverabreichungen aufgeteilt. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,1 bis 50 mg, vorzugsweise 0,5 bis 10 mg Wirkstoff der allgemeinen Formel I oder eines pharmakologisch annehmbaren Salzes davon pro Dosis.

Die folgenden Beispiele 1 bis 11 betreffen die Herstellung von Verbindungen der allgemeinen Formel I, die Beispiele A bis H betreffen die Herstellung von Zubereitungen der Verbindungen der allgemeinen Formel I.

### Beispiel 1:

### Thien-2-yl-aminoessigsäure-tert.butylester

Das Gemisch aus 12,6 g Thien-2-yl-aminoessigsäure, 300 ml Dimethoxyethan, 15 ml Schwefelsäure und 100 ml Isobuten wird 5 h bei Raumtemperatur im Autoklaven gerührt und in ein Gemisch bestehend aus 800 ml Ether und 800 ml 1 N Natronlauge gegossen. Die etherische Phase wird abgetrennt, die wäßrige Phase noch zweimal mit je 200 ml Ether ausgeschüttelt. Nach dem Trocknen der Etherphasen über MgSO₄ und dem Einengen des Lösungsmittels verbleibt ein gelblicher, öliger Rückstand.
Ausbeute: 6,8 g Öl

### Beispiel 2:

### Thien-2-yl-aminoessigsäuremethylester HCl

In die Lösung von 6,8 g Thien-2-yl-aminoessigsäure in 70 ml Methanol wird HCl-Gas eingeleitet und die Mischung 6 h auf 50°C erwärmt. Das überschüssige Methanol und die Salzsäure werden im Vakuum abgezogen und der feste Rückstand mit Methanol gewaschen und im Vakuum getrocknet.
Ausbeute: 5,9 g FP.: 179°C

### Beispiel 3:

### Thien-2-yl-N-methoxycarbonylmethyl-aminoessigsäure-tert.butylester

Das Gemisch aus 6,6 g Thien-2-yl-aminoessigsäure-tert.butylester (Beispiel 1), 3,3 g Triethylamin, 5,0 g Bromessigsäuremethylester und 40 ml Dimethoxyethan wird 4 h am Rückfluß gekocht. Die flüchtigen Anteile werden im Vakuum entfernt, der Rest mit 250 ml Wasser verrührt und das Produkt mit Essigester ausgeschüttelt. Nach dem Trocknen über Na₂SO₄ wird Aktivkohle zugesetzt, kurz aufgekocht und filtriert. Nach dem Verdampfen des Lösungsmittels verbleibt ein öliger Rückstand.
Ausbeute: 7,4 g Öl

### Beispiel 4:

### Thien-2-yl-N-aminocarbonylmethyl-aminoessigsäure-tert.-butylester

Die Mischung aus 7,4 g der Verbindung aus Beispiel 3 und 50 ml konz. methanolischer Ammoniak wird 15 h bei Raumtemperatur stehen gelassen. Das Lösungsmittel wird abgezogen, der Rückstand in heißem Essigester mit Aktivkohle behandelt und die Mischung filtriert. Nach dem Verdampfen des Lösungsmittels wird der verbleibende Rückstand mit Diethylether verrührt, abgesaugt und getrocknet.
Ausbeute: 3,2 g Fp.: 115 - 117°C

### Beispiel 5:

### Thien-2-yl-N-aminocarbonylmethyl-aminoessigsäure

In der Lösung von 2,4 g der Verbindung aus Beispiel 4 in 25 ml Eisessig wird unter Kühlung HCl-Gas bis zur Sättigung eingeleitet. Die Mischung läßt man unter Rühren Raumtemperatur erreichen. Der sich bildende Niederschlag wird abgesaugt, mit wenig Methanol gewaschen und getrocknet.
Ausbeute: 2,0 g Fp.: 206°C (Zers.)

### Beispiel 6:

### Thien-2-yl-aminoessigsäureamid

### a) Thien-2-yl-glyoxyl-säure-amid

In die Lösung von 73,6 g Thien-2-yl-glyoxylsäureethylester werden unter Kühlung 13,6 g Ammoniak eingeleitet und die erhaltene Mischung 2 h bei Raumtemperatur gerührt. Das Lösungsmittel wird im Vakuum abgezogen und der Rückstand aus Wasser:Ethanol (80:20) umkristallisiert.
Ausbeute: 40,3 g Fp.: 86 - 88°C

### b) Thien-2-yl-hydroximino-essigsäure-amid

Die Mischung aus 15,5 g Thien-2-yl-glyoxylsäureamid, 75 ml Wasser, 75 ml Ethanol, 10,4 g Hydroxylaminhydrochlorid und 24,6 g Natriumacetat wird 5 h bei 40°C gerührt, die flüchtigen Anteile werden im Vakuum abgezogen und der Rückstand in Wasser aufgenommen. Das Produkt wird mit Essigester extrahiert, die organische Phase getrocknet und eingeengt. Der Rückstand wird aus wenig Essigester umkristallisiert.
Ausbeute: 5,2 g Fp.: 146 - 149°C

### c) Thien-2-yl-aminoessigsäureamid

Die Mischung aus 5,1 g Thien-2-yl-hydroximinoessigsäureamid und 180 ml Methanol wird in Gegenwart von Raney-Nickel bei 100°C/50 bar hydriert. Nach Beendigung der Wasserstoffaufnahme wird vom Raney Nickel abgesaugt, das Filtrat eingeengt und der Rückstand aus Diethylether umkristallisiert.
Ausbeute: 2,6 g Fp.: 82 - 84°C

### Beispiel 7:

### Thien-2-yl-N-tert.butoxycarbonylmethyl-aminoessigsäureamid

Die Lösung von 9,4 g Thien-2-yl-aminoessigsäureamid (Beispiel 6), 12,9 g Bromessigsäuretert.butylester und 6,7 g Triethylamin in 100 ml Dimethoxyethan wird 16 h zum Sieden erhitzt. Der nach dem Einengen verbleibende Rückstand wird in Wasser/Essigester verteilt. Die Essigesterphase wird getrocknet, mit Aktivkohle aufgekocht, filtriert und eingeengt. Der Rückstand wird aus Essigester umkristallisiert.
Ausbeute: 3,4 g Fp.: 94 - 95°C

### Beispiel 8:

### Thien-2-yl-N-hydroxycarbonylmethyl-aminoessigsäure-amidhydrochlorid

In die Suspension von 3,2 g der Verbindung aus Beispiel 7 in 30 ml Eisessig wird unter Rühren und Kühlung bis zur Sättigung HCl-Gas eingeleitet. Es wird 1 h nachgerührt und die flüchtigen Anteile im Vakuum entfernt. Der Rückstand wird aus Ether umkristallisiert.
Ausbeute: 2,9 g Fp.: 208°C (Zers.)

### Beispiel 9:

### Thien-2-yl-N-aminoacetyl-aminoessigsäure-hydrochlorid

### a) Thien-2-yl-N-tert.butoxycarbonylaminoacetyl-aminoessigsäure-tert.-butylester

N-tert.Butoxycarbonyl-glycin (4,3 g) und 2,5 g Triethylamin werden in 240 ml Tetrahydrofuran gelöst und auf -20°C abgekühlt. Dann werden 2,9 g Trimethylessigsäurechlorid zugetropft und 30 min bei -20°C nachgerührt. Zu dieser Mischung wird die Lösung von 7 g der Verbindung aus Beispiel 1 und 2,5 g Triethylamin in 20 ml Dimethylformamid getropft. Das Kältebad wird entfernt, und die Mischung wird 2 h bei Raumtemperatur gerührt. Der Niederschlag wird abgesaugt, das Filtrat eingeengt. Der ölige Rückstand wird in Essigester gelöst, zweimal mit je 100 ml Wasser, einmal mit 100 ml 5%iger Natriumbicarbonatlösung und einmal mit 100 ml 1 N Zitronensäure gewaschen, über Magnesiumsulfat getrocknet. Nach dem Entfernen des Lösungsmittels verbleibt ein öliges Produkt.
Ausbeute: 6,4 g Öl

### b) Thien-2-yl-N-aminoacetyl-aminoessigsäure-hydrochlorid

Die Lösung von 9 g der Verbindung aus Beispiel 9a) in 50 ml Eisessig wird mit HCl-Gas unter Eiskühlung gesättigt. Unter Rühren läßt man Raumtemperatur erreichen, wobei sich ein Niederschlag bildet, der abgesaugt wird. Der Feststoff wird in wenig Methanol aufgenommen und mit etwas Aktivkohle aufgekocht. Nach dem Filtrieren wird das Produkt durch Zugabe von Diethylether ausgefällt und abgesaugt.
Ausbeute: 2,2 g Fp.: 197°C (Zers.)

### Beispiel 10:

### Thien-2-yl-N-ethoxycarbonylmethyl-aminoessigsäureamid

wird analog dem Beispiel 7 aus 15,6 g Thien-2-yl-aminoessigsäureamid und 16,7 g Bromessigsäureethylester erhalten.
Ausbeute: 6,8 g Fp.: 120 - 122°C

### Beispiel 11:

### N-(Thien-2-yl-aminoacetyl-glycin

### a) N-(Thien-2-yl-hydroximino-acetyl)-glycin

Zur Mischung von 22 g Thien-2-yl-glyoxylsäure-N-hydroxicarbonylmethylamid (hergestellt aus Aminoessigsäure und Thien-2-yl-glyoxylsäureethylester, Fp. 168-170°C), 15 ml Wasser, 50 ml Methanol und 9,5 g Hydroxylamin-hydrochlorid wird portionsweise 17,6 g Natriumhydroxid in Pulverform gegeben. Die Temperatur wird durch Kühlung auf 40 - 50°C gehalten und die Mischung dann bei Raumtemperatur 2 h nachgerührt. Mit konz. Salzsäure wird sauer gestellt und anschließend die flüchtigen Anteile am Rotationsverdampfer abgezogen. Der Rückstand wird mit 300 ml Ethanol aufgekocht, mit Aktivkohle filtriert und das Filtrat im Vakuum eingeengt. Der Rückstand wird mit Diethylether verrührt und abgesaugt.
Ausbeute: 7,0 g Fp. 175 - 177°C

### b) N-(Thien-2-yl-aminoacetyl)-glycin

Die Lösung aus 11,4 g Thien-2-yl-hydroximino-acetylglycin und 7 ml Ammoniak in 100 ml Methanol wird mit Raney-Nickel als Katalysator bis zur Beendigung der Wasserstoffaufnahme bei 100°C/50 bar im Autoklaven hydriert. Vom Katalysator wird abgesaugt und das Filtrat im Vakuum eingeengt. Der Rückstand wird in 100 ml Ethanol suspendiert und erhitzt und mit gerade soviel Wasser versetzt, daß eine klare Lösung entsteht. Dann wird Aktivkohle zugesetzt, filtriert und im Eisbad abgekühlt. Der Niederschlag wird abgesaugt und getrocknet.
Ausbeute: 7,8 g Fp.: 208°C (Zers.)

### Beispiel A

Emulsionen mit 3 mg Wirkstoff per 5ml können nach folgender Rezeptur hergestellt werden:

| | |
|---|---|
| Wirkstoff | 0,06g |
| Neutralöl | q.s. |
| Natriumcarboxymethylzellulose | 0,6g |
| Polyoxyethylenstearat | q.s. |
| Reinglyzerin | 0,2 bis 2g |
| Aromastoffe | q.s. |
| Wasser (entmineralisiert oder destilliert) | ad 100ml |

### Beispiel B

Tabletten können nach folgender Formulierung hergestellt werden:

| | |
|---|---|
| Wirkstoff | 2mg |
| Lactose | 60mg |
| Maisstärke | 30mg |
| lösliche Stärke | 4mg |
| Magnesiumstearat | 4mg |
| | 10̅0̅m̅g̅ |

### Beispiel C

Für die Herstellung von Weichgelatinekapseln mit 5mg Wirkstoff pro Kapsel eignet sich die folgende Zusammensetzung:

| | |
|---|---|
| Wirkstoff | 5mg |
| Mischung von Triglyzeriden aus Kokosöl | 150mg |
| Kapselinhalt | 1̅5̅5̅m̅g̅ |

### Beispiel D

Für die Herstellung von Dragees eignet sich folgende Formulierung

| | |
|---|---|
| Wirkstoff | 3mg |
| Maisstärke | 100mg |
| Lactose | 55mg |
| sec Calciumphosphat | 30mg |
| lösliche Stärke | 3mg |
| Magnesiumstearat | 5mg |
| kolloidale Kieselsäure | 4mg |
| | 2̅0̅0̅m̅g̅ |

### Beispiel E

Dragees, enthaltend einen erfindungsgemäßen Wirkstoff und einen anderen therapeutisch wirksamen Stoff:

| | |
|---|---|
| Wirkstoff | 6mg |
| Propranolol | 40mg |
| Milchzucker | 90mg |
| Maisstärke | 90mg |
| sec Calciumphosphat | 34mg |
| lösliche Stärke | 3mg |
| Magnesiumstearat | 3mg |
| kolloidale Kieselsäure | 4mg |
| | 270mg |

### Beispiel F

Dragees, enthaltend einen erfindungsgemäßen Wirkstoff und einen anderen therapeutisch wirksamen Stoff:

| | |
|---|---|
| Wirkstoff | 5mg |
| Pirlindol | 5mg |
| Milchzucker | 60mg |
| Maisstärke | 90mg |
| sec Calciumphosphat | 30mg |
| lösliche Stärke | 3mg |
| Magnesiumstearat | 3mg |
| kolloidale Kieselsäure | 4mg |
| | 2̅0̅0̅m̅g̅ |

### Beispiel G

Kapseln, enthaltend einen erfindungsgemäßen Wirkstoff und einen anderen therapeutisch wirksamen Stoff:

| | |
|---|---|
| Wirkstoff | 5mg |
| Nicergolin | 5mg |
| Maisstärke | 185mg |
| | 1̅9̅5̅m̅g̅ |

### Beispiel H

Injektionslösungen mit 1mg Wirkstoff pro ml können nach folgender Rezeptur hergestellt werden:

| | |
|---|---|
| Wirkstoff | 1,0mg |
| Polyethylenglykol 400 | 0,3mg |
| Natriumchlorid | 2,7mg |
| Wasser zu Injektionszwecken auf | 1 ml |

## Patentansprüche (Patentansprüche für folgende(n) Vertragsstaat(en): AT, BE, CH, DE, DK, FR, GB, IT, LI, NL, SE)

1. Thienylessigsäurederivate der allgemeinen Formel I 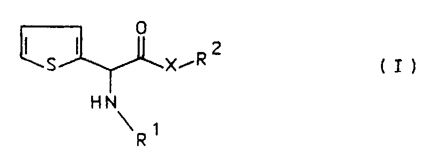 worin
X -O- oder -NH-;
R¹ Wasserstoff, die Gruppe -CH₂-CO-R³ oder die Gruppe -CO-CH₂-NH₂;
R² Wasserstoff, (C₁-C₄)-Alkyl oder den Rest R¹ und
R³ (C₁-C₄)-Alkoxy, Hydroxy oder Amino bedeuten, wobei R¹ und R² nicht gleichzeitig für Wasserstoff stehen können, wenn X = O bedeutet,
sowie ihre pharmazeutisch verträglichen Salze.

2. Thienylessigsäurederivate gemäß Anspruch 1, dadurch gekennzeichnet, daß
X -O-;
R¹ die Gruppe -CH₂-CO-R³;
R² Wasserstoff oder (C₁-C₄)-Alkyl und
R³ (C₁-C₄)-Alkoxy, Hydroxy oder Amino
bedeuten.

3. Thienylessigsäurederivate gemäß Anspruch 1, dadurch gekennzeichnet, daß
X -NH-;
R¹ die Gruppe -CH₂-CO-R³;
R² Wasserstoff und
R³ (C₁-C₄)-Alkoxy oder Hydroxy
bedeuten.

4. Thien-2-yl-N-aminocarbonylmethyl-aminoessigsäure.

5. Thien-2-yl-N-hydroxycarbonylmethyl-aminoessigsäureamid-hydrochlorid.

6. Verfahren zur Herstellung von Thienylessigsäurederivaten gemäß den Ansprüchen 1 bis 5, dadurch gekennzeichnet, daß
A) eine Verbindung der allgemeinen Formel II 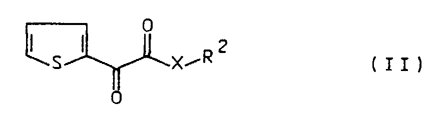 worin X und R² wie im Anspruch 1 definiert sind, mit Hydroxylamin zum Oxim und dieses durch Reduktion zur Verbindung der allgemeinen Formel Ia 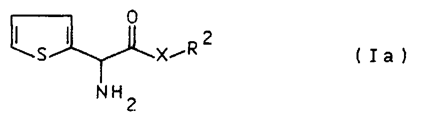 umgesetzt wird, wobei letztere gegebenenfalls
a) durch Alkylierung mit Halogenessigsäureestern der allgemeinen Formel III 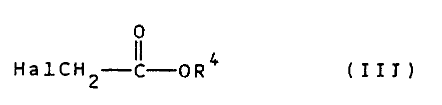 worin
Hal Halogen, bevorzugt Brom, und
R⁴ (C₁-C₄)-Alkyl
bedeuten, zu Verbindungen der allgemeinen Formel Ib 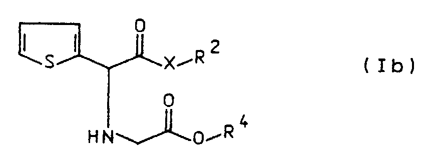 und diese gegebenenfalls durch Hydrolyse oder Ammonolyse mit Ammoniak zu Verbindungen der allgemeinen Formel Ic 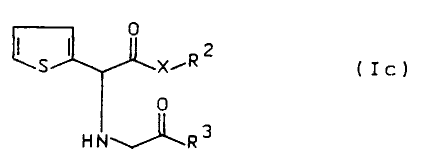 worin R³ -OH oder -NH₂ bedeutet, umgesetzt werden können, oder
b) durch Umsetzung mit dem gemischten Anhydrid der Formel IV 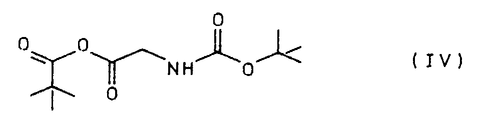 und anschließender Hydrolyse in die Verbindungen der Formel Id 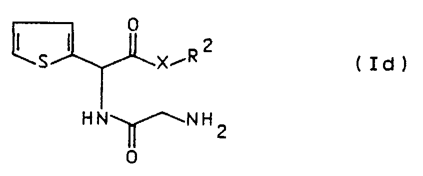 überführt werden können oder daß
B) 2-Thienylglycin der Formel V 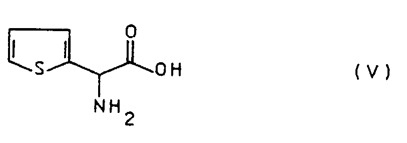 zu Verbindungen der allgemeinen Formel Ia verestert bzw-. amidiert wird und diese gegebenenfalls, wie oben unter A) beschrieben, weiter umgesetzt werden, wobei die Verbindungen der allgemeinen Formeln Ia bis Id gegebenenfalls noch in ein Salz überführt werden.

7. Verwendung eines Thienylessigsäurederivats gemäß einem oder mehreren der Ansprüche 1 bis 5 zur Herstellung eines pharmazeutischen Präparates zur Bekämpfung und Vorbeugung von Erkrankungen, die durch Einschränkung der Gehirnfunktion bedingt sind, oder zur Bekämpfung von cerebralen Alterungsvorgängen.

8. Pharmazeutisches Präparat, dadurch gekennzeichnet, daß es als Wirkstoff ein oder mehrere Thienylessigsäurederivate gemäß einem oder mehreren der Ansprüche 1 bis 5 oder ein pharmazeutisch verträgliches Salz davon zusammen mit einem pharmazeutisch annehmbaren Trägerstoff und gegebenenfalls pharmazeutisch annehmbaren Zusatzstoffen und gegebenenfalls noch einen oder mehrere andere pharmakologische Wirkstoffe enthält.

9. Verfahren zur Herstellung eines pharmazeutischen Präparates gemäß Anspruch 8, dadurch gekennzeichnet, daß ein oder mehrere Thienylessigsäurederivate gemäß einem oder mehreren der Ansprüche 1 bis 5 oder ein pharmazeutisch verträgliches Salz davon durch Mischen mit geeigneten neten pharmazeutisch zulässigen Träger- und Zusatzstoffen und gegebenenfalls noch einer oder mehreren anderen pharmazeutisch wirksamen Substanzen in eine zur Verabreichung geeignete Form gebracht werden.

## Patentansprüche (Patentansprüche für folgende(n) Vertragsstaat(en): ES, GR)

1. Verfahren zur Herstellung von Thienylessigsäurederivaten der allgemeinen Formel I 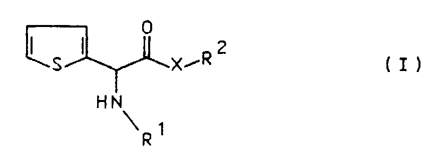 worin
X -O- oder -NH-;
R¹ Wasserstoff, die Gruppe -CH₂-CO-R³ oder die Gruppe -CO-CH₂-NH₂;
R² Wasserstoff, (C₁-C₄)-Alkyl oder den Rest R¹ und
R³ (C₁-C₄)-Alkoxy, Hydroxy oder Amino bedeuten, wobei R¹ und R² nicht gleichzeitig für Wasserstoff stehen können, wenn X = O bedeutet,
sowie ihre pharmazeutisch verträglichen Salze, dadurch gekennzeichnet, daß
A) eine Verbindung der allgemeinen Formel II 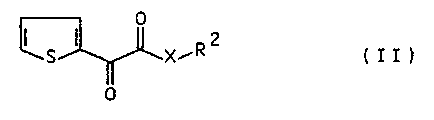 worin X und R² wie im Anspruch 1 definiert sind, mit Hydroxylamin zum Oxim und dieses durch Reduktion zur Verbindung der allgemeinen Formel Ia 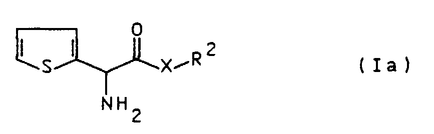 umgesetzt wird, wobei letztere gegebenenfalls
a) durch Alkylierung mit Halogenessigsäureestern der allgemeinen Formel III 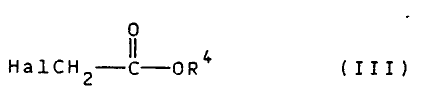 worin
Hal Halogen, bevorzugt Brom, und
R⁴ (C₁-C₄)-Alkyl
bedeuten, zu Verbindungen der allgemeinen Formel Ib 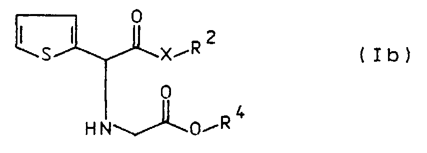 und diese gegebenenfalls durch Hydrolyse oder Ammonolyse mit Ammoniak zu Verbindungen der allgemeinen Formel Ic 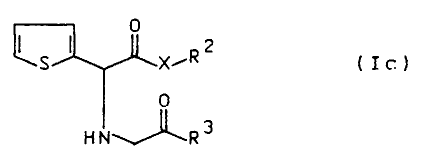 worin R³ -OH oder -NH₂ bedeutet, umgesetzt werden können, oder
b) durch Umsetzung mit dem gemischten Anhydrid der Formel IV 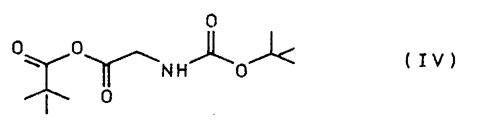 und anschließender Hydrolyse in die Verbindungen der Formel Id 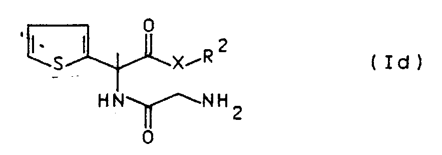 überführt werden können oder daß
B) 2-Thienylglycin der Formel V 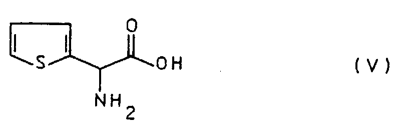 zu Verbindungen der allgemeinen Formel Ia verestert bzw-. amidiert wird und diese gegebenenfalls, wie oben unter A) beschrieben, weiter umgesetzt werden, wobei die Verbindungen der allgemeinen Formeln Ia bis Id gegebenenfalls noch in ein Salz überführt werden.

2. Verfahren gemäß Anspruch 1, dadurch gekennzeichnet, daß eine Verbindung der allgemeinen Formel I hergestellt wird, worin
X -O-;
R¹ die Gruppe -CH₂-CO-R³;
R² Wasserstoff oder (C₁-C₄)-Alkyl und
R³ (C₁-C₄)-Alkoxy, Hydroxy oder Amino
bedeuten.

3. Verfahren gemäß Anspruch 1, dadurch gekennzeichnet, daß eine Verbindung der allgemeinen Formel I hergestellt wird, worin
X -NH-;
R¹ die Gruppe -CH₂-CO-R³;
R² Wasserstoff und
R³ (C₁-C₄)-Alkoxy oder Hydroxy
bedeuten.

4. Verfahren gemäß Anspruch 1, dadurch gekennzeichnet, daß Thien-2-yl-N-aminocarbonylmethyl-aminoessigsäure hergestellt wird.

5. Verfahren gemäß Anspruch 1, dadurch gekennzeichnet, daß Thien-2-yl-N-hydroxycarbonylmethyl-aminoessigsäure-amidhydrochlorid hergestellt wird.

6. Verwendung eines nach einem der Verfahren der Ansprüche 1 bis 5 hergestllten Thienylessigsäurederivats der allgemeinen Formel I zur Herstellung eines pharmazeutischen Präparates zur Bekämpfung und Vorbeugung von Erkrankungen, die durch Einschränkung der Gehirnfunktion bedingt sind, oder zur Bekämpfung von cerebralen Alterungsvorgängen.

7. Verfahren zur Herstellung eines pharmazeutischen Präparates, dadurch gekennzeichnet, daß ein oder mehrere nach den Verfahren der Ansprüche 1 bis 5 hergestellten Thienylessigsäurederivate der allgemeinen Formel I oder ein pharmazeutisch verträgliches Salz davon durch Mischen mit geeigneten pharmazeutisch zulässigen Träger- und Zusatzstoffen und gegebenenfalls noch einer oder mehreren anderen pharmazeutisch wirksamen Substanzen in eine zur Verabreichung geeignete Form gebracht werden.

## Claims (Claims for the following Contracting State(s): AT, BE, CH, DE, DK, FR, GB, IT, LI, NL, SE)

1. Thienylacetic acid derivatives of the general formula I 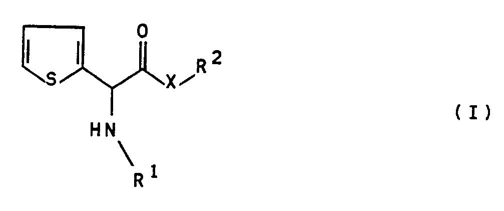 in which
X denotes -O- or -NH-;
R¹ denotes hydrogen, the group -CH₂-CO-R³ or the group -CO-CH₂-NH₂;
R² denotes hydrogen, (C₁-C₄)-alkyl or the radical R¹ and
R³ denotes (C₁-C₄)-alkoxy, hydroxyl or amino, it not being possible for both R¹ and R² to represent hydrogen when X denotes O, as well as the pharmaceutically tolerated salts thereof.

2. Thienylacetic acid derivatives according to Claim 1, characterized in that
X denotes -O-;
R¹ denotes the group -CH₂-CO-R³;
R² denotes hydrogen or (C₁-C₄)-alkyl and
R³ denotes (C₁-C₄)-alkoxy, hydroxyl or amino.

3. Thienylacetic acid derivatives according to Claim 1, characterized in that
X denotes -NH-;
R¹ denotes the group -CH₂-CO-R³;
R² denotes hydrogen and
R³ denotes (C₁-C₄)-alkoxy or hydroxyl.

4. 2-Thienyl-N-aminocarbonylmethylaminoacetic acid.

5. 2-Thienyl-N-hydroxycarbonylmethylaminoacetamide hydrochloride.

6. Process for the preparation of thienylacetic acid derivatives according to Claims 1 to 5, characterized in that A) a compound of the general formula II 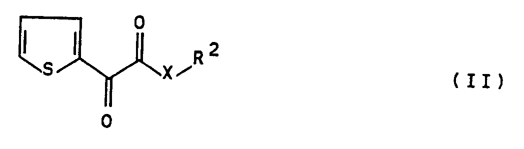 in which X and R² are as defined in Claim 1, is converted with hydroxylamine into the oxime and the latter is reduced to the compound of the general formula Ia 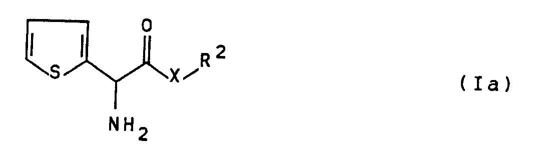 it being possible to convert the latter where appropriate
a) by alkylation with halogenoacetic esters of the general formula III 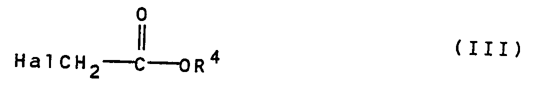 in which Hal denotes halogen, preferably bromine, and R⁴ denotes (C₁-C₄)-alkyl, into compounds of the general formula Ib 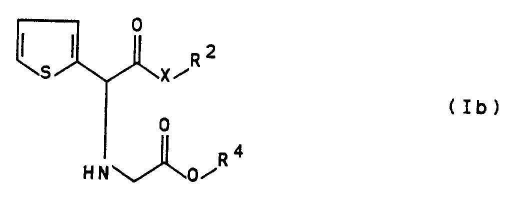 and the latter where appropriate by hydrolysis or ammonolysis with ammonia into compounds of the general formula Ic 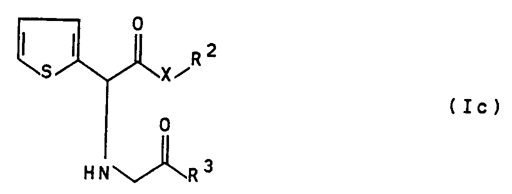 in which R³ denotes -OH or -NH₂, or
b) by reaction with the mixed anhydride of the formula IV 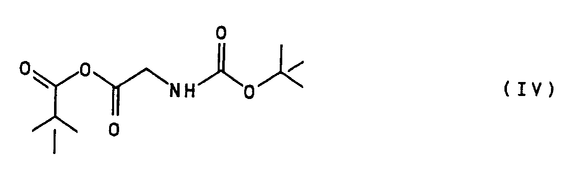 and subsequent hydrolysis into the compounds of the formula Id 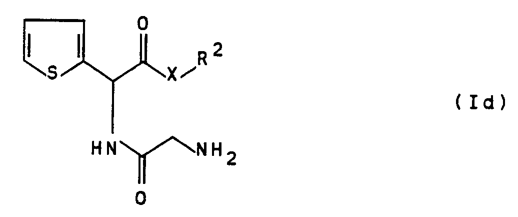 or in that
B) 2-thienylglycine of the formula V 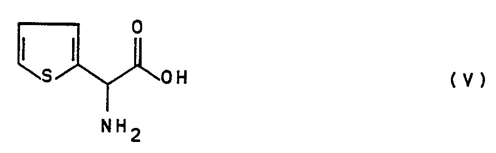 is esterified or amidated to give compounds of the general formula Ia, and the latter are, where appropriate, further reacted as described above under A), it also being possible where appropriate to convert the compounds of the general formula Ia to Id into a salt.

7. Use of a thienylacetic acid derivative according to one or more of Claims 1 to 5 for preparing a pharmaceutical preparation for controlling and preventing disorders based on impairment of brain function or for controlling cerebral ageing processes.

8. Pharmaceutical product, characterized in that it contains as active compound one or more thienylacetic acid derivatives according to one or more of Claims 1 to 5 or a pharmaceutically tolatered salt thereof together with a pharmaceutically acceptable vehicle and, where appropriate, pharmaceutically acceptable additives and, where appropriate, also one or more other pharmacological active compounds.

9. Process for the preparation of a pharmaceutical product according to Claim 8, characterized in that one or more thienylacetic acid derivatives according to one or more of Claims 1 to 5 or a pharmaceutically tolerated salt thereof is converted into a form suitable for administration by mixing with suitable pharmaceutically acceptable vehicles and additives and, where appropriate, also one or more other pharmaceutically active substances.

## Claims (Claims for the following Contracting State(s): ES, GR)

1. Process for preparing thienylacetic acid derivatives of the general formula I 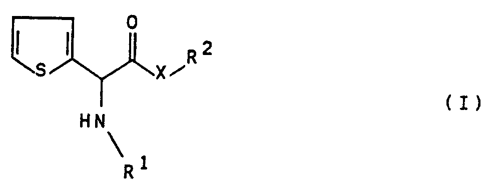 in which
X denotes -O- or -NH-;
R¹ denotes hydrogen, the group -CH₂-CO-R³ or the group -CO-CH₂-NH₂;
R² denotes hydrogen, (C₁-C₄)-alkyl or the radical R¹ and
R³ denotes (C₁-C₄)-alkoxy, hydroxyl or amino, it not being possible for both R¹ and R² to represent hydrogen when X denotes O, as well as the pharmaceutically tolerated salts thereof, characterized in that A) a compound of the general formula II 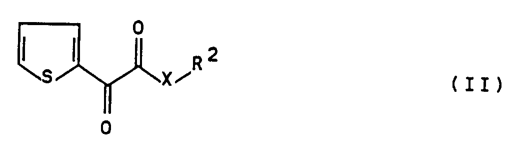 in which X and R² are as defined in Claim 1, is converted with hydroxylamine into the oxime and the latter is reduced to the compound of the general formula Ia 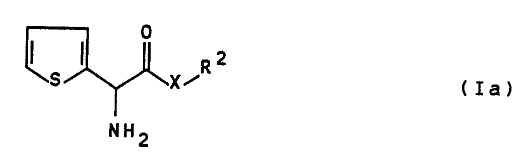 it being possible to convert the latter where appropriate
a) by alkylation with halogenoacetic esters of the general formula III 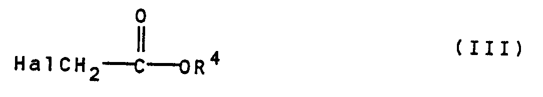 in which Hal denotes halogen, preferably bromine, and R⁴ denotes (C₁-C₄)-alkyl, into compounds of the general formula Ib 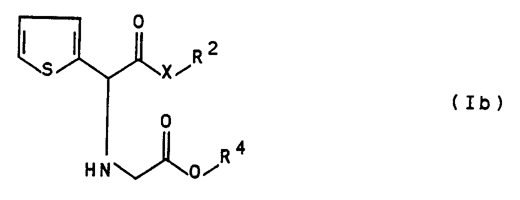 and the latter where appropriate by hydrolysis or ammonolysis with ammonia into compounds of the general formula Ic 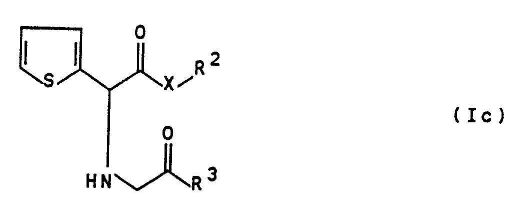 in which R³ denotes -OH or -NH₂, or
b) by reaction with the mixed anhydride of the formula IV 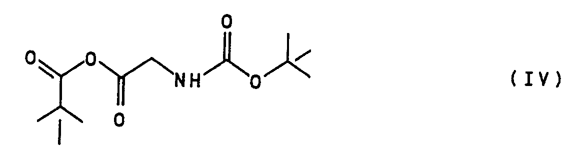 and subsequent hydrolysis into the compounds of the formula Id 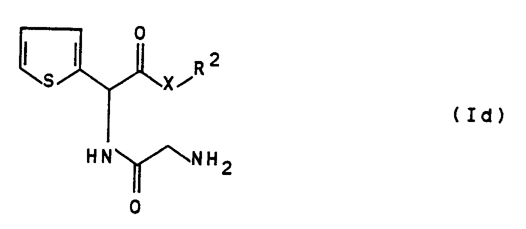 or in that
B) 2-thienylglycine of the formula V 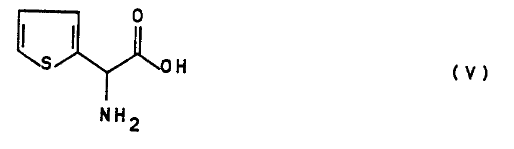 is esterified or amidated to give compounds of the general formula Ia, and the latter are, where appropriate, further reacted as described above under A), it also being possible where appropriate to convert the compounds of the general formula Ia to Id into a salt.

2. Process according to Claim 1, characterized in that a compound of the general formula I is prepared wherein
X denotes -O-;
R¹ denotes the group -CH₂-CO-R³;
R² denotes hydrogen or (C₁-C₄)-alkyl and
R³ denotes (C₁-C₄)-alkoxy, hydroxyl or amino.

3. Process according to Claim 1, characterized in that a compound of the general formula I is prepared wherein
X denotes -NH-;
R¹ denotes the group -CH₂-CO-R³;
R² denotes hydrogen and
R³ denotes (C₁-C₄)-alkoxy or hydroxyl.

4. Process according to Claim 1, characterized in that 2-thienyl-N-aminocarbonylmethylaminoacetic acid is prepared.

5. Process according to Claim 1, characterized 2-thienyl-N-hydroxycarbonylmethylaminoacetamide hydrochloride is prepared.

6. Use of a thienylacetic acid derivative of the general formula I prepared according to a process of Claims 1 to 5 for preparing a pharmaceutical preparation for controlling and preventing disorders based on impairment of brain function or for controlling cerebral ageing processes.

7. Process for the preparation of a pharmaceutical product, characterized in that one or more thienylacetic acid derivatives of the general formula I prepared according to a process of Claims 1 to 5 or a pharmaceutically tolerated salt thereof is converted into a form suitable for administration by mixing with suitable pharmaceutically acceptable vehicles and additives and, where appropriate, also one or more other pharmaceutically active substances.

## Revendications (Revendications pour l'(les) Etat(s) contractant(s) suivant(s): AT, BE, CH, DE, DK, FR, GB, IT, LI, NL, SE)

1. Dérivés de l'acide thiénylacétique de formule générale I 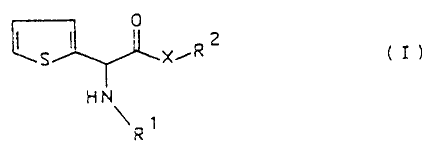 dans laquelle
X est -O- ou -NH- ;
R¹ est un hydrogène, le groupe -CH₂-CO-R³ ou le groupe -CO-CH₂-NH₂ ;
R² est un hydrogène, un radical alkyle en C₁-C₄ ou le radical R¹, et
R³ est un radical alcoxy en C₁-C₄, hydroxy ou amino, R¹ et R² ne pouvant être simultanément un hydrogène quand X = O, et leurs sels pharmaceutiquement acceptables.

2. Dérivés de l'acide thiénylacétique selon la revendication 1, caractérisés en ce que :
X est -O- ;
R¹ est le groupe -CH₂-CO-R³ ;
R² est un hydrogène ou un radical alkyle en C₁-C₄, et
R³ est un radical alcoxy en C₁-C₄, hydroxy ou amino.

3. Dérivés de l'acide thiénylacétique selon la revendication 1, caractérisés en ce que :
X est -NH- ;
R¹ est le groupe -CH₂-CO-R³ ;
R² est un hydrogène et
R³ est un radical alcoxy en C₁-C₄ ou hydroxy.

4. Acide thiényl-2 N-aminocarbonylméthylaminoacétique.

5. Chlorhydrate du thiényl-2 N-hydroxycarbonylméthylaminoacétamide.

6. Procédé pour préparer des dérivés de l'acide thiénylacétique selon les revendications 1 à 5, caractérisé en ce que
A) on fait réagir un composé de formule générale II 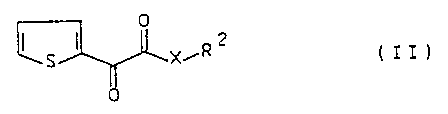 dans laquelle X et R² ont les définitions données dans la revendication 1, avec de l'hydroxylamine pour obtenir l'oxime, et on fait réagir ce dernier par réduction pour obtenir le composé de formule générale Ia 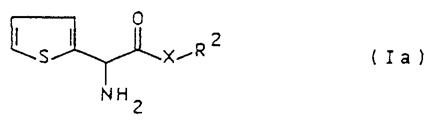 ce dernier pouvant éventuellement
a) être mis à réagir par alkylation avec des halogénoacétates de formule générale III 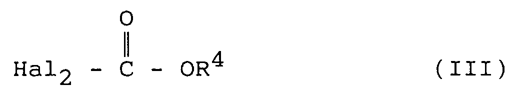 dans laquelle
Hal est un halogène, de préférence le brome, et
R⁴ est un radical alkyle en C₁-C₄,
pour obtenir des composés de formule générale Ib 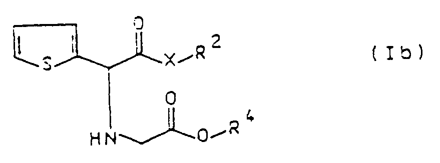 ce dernier pouvant éventuellement être mis à réagir par hydrolyse ou ammonolyse avec de l'ammoniac pour obtenir des composés de formule générale Ic 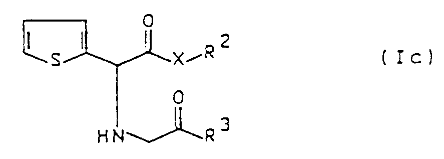 dans laquelle R³ est -OH ou -NH₂, ou bien
b) on peut, par réaction avec l'anhydride mixte de formule IV 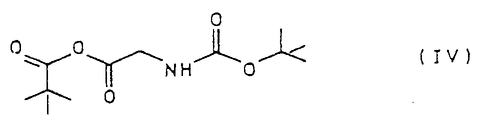 puis hydrolyse, les convertir en les composés de formule Id 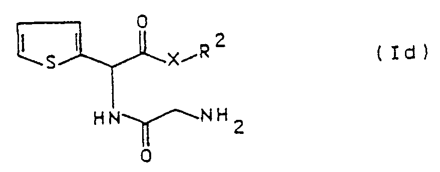 ou bien en ce que
B) on estérifie ou amide la thiényl-2 glycine de formule V 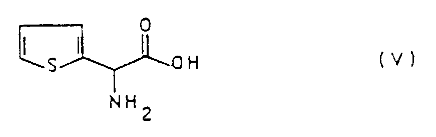 pour obtenir des composés de formule générale Ia, ces derniers étant éventuellement, comme décrit ci-dessus en A), soumis à des réactions plus poussées, les composés ayant les formules générales Ia à Id étant éventuellement encore convertis en un sel.

7. Utilisation d'un dérivé de l'acide thiénylacétique selon l'une ou plusieurs des revendications 1 à 5 pour réaliser une préparation pharmaceutique destinée à lutter contre les maladies dues à une restriction de la fonction cérébrale, et à les prévenir, ou à lutter contre les processus de vieillissement cérébral.

8. Préparation pharmaceutique, caractérisée en ce qu'elle contient en tant que principe actif un ou plusieurs dérivés de l'acide thiénylacétique selon l'une ou plusieurs des revendications 1 à 5 ou l'un de leurs sels pharmaceutiquement acceptables, en même temps qu'un excipient pharmaceutiquement acceptable et éventuellement des additifs pharmaceutiquement acceptables, et éventuellement encore un ou plusieurs autres principes actifs pharmacologiques.

9. Procédé pour réaliser une préparation pharmaceutique selon la revendication 8, caractérisé en ce qu'on transforme en une forme administrable un ou plusieurs dérivés de l'acide thiénylacétique selon l'une ou plusieurs des revendications 1 à 5 ou un de leurs sels pharmaceutiquement acceptables, par mélange à des adjuvants et additifs appropriés, pharmaceutiquement acceptables, et éventuellement encore d'une ou plusieurs autres substances à effet pharmaceutique.

## Revendications (Revendications pour l'(les) Etat(s) contractant(s) suivant(s): ES, GR)

1. Procédé pour préparer des dérivés de l'acide thiénylacétique de formule générale I 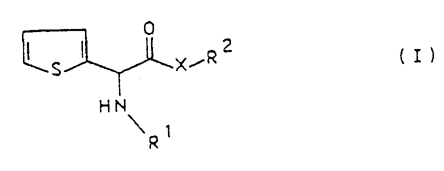 dans laquelle
X est -O- ou -NH- ;
R¹ est un hydrogène, le groupe -CH₂-CO-R³ ou le groupe -CO-CH₂-NH₂ ;
R² est un hydrogène, un radical alkyle en C₁-c₄ ou le radical R¹, et
R³ est un radical alcoxy en C₁-C₄, hydroxy ou amino, R¹ et R² ne pouvant être simultanément un hydrogène quand X = O, ainsi que leurs sels pharmaceutiquement acceptables, caractérisé en ce que
A) on fait réagir un composé de formule générale II 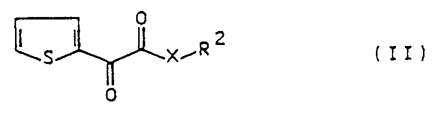 dans laquelle X et R² ont les définitions données dans la revendication 1, avec de l'hydroxylamine pour obtenir l'oxime, et on fait réagir ce dernier par réduction pour obtenir le composé de formule générale Ia 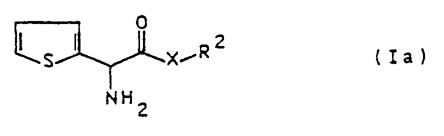 ce dernier pouvant éventuellement
a) être mis à réagir par alkylation avec des halogénoacétates de formule générale III 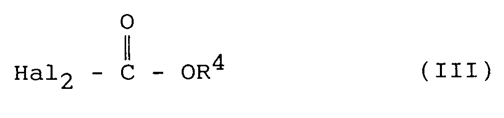 dans laquelle
Hal est un halogène, de préférence le brome, et
R⁴ est un radical alkyle en C₁-C₄,
pour obtenir des composés de formule générale Ib 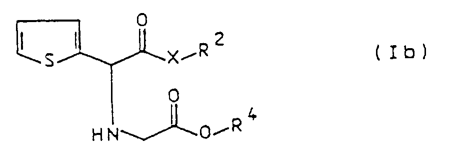 ce dernier pouvant éventuellement être mis à réagir par hydrolyse ou ammonoloyse avec de l'ammoniac pour obtenir des composés de formule générale Ic 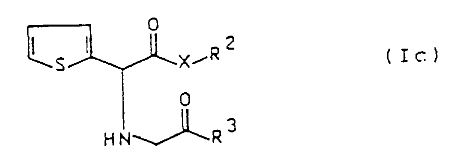 dans laquelle R³ est -OH ou -NH₂, ou bien
b) on peut, par réaction avec l'anhydride mixte de formule IV 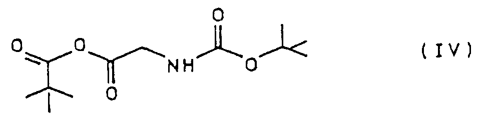 puis hydrolyse, les convertir en les composés de formule Id 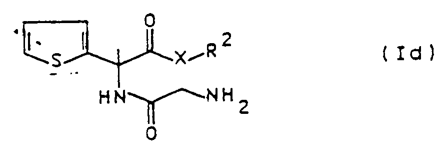 ou bien en ce que
B) on estérifie ou amide la thiényl-2 glycine de formule V 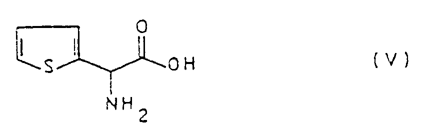 pour obtenir des composés de formule générale Ia, ces derniers étant éventuellement, comme décrit ci-dessus en A), soumis à des réactions plus poussées, les composés ayant les formules générales Ia à Id étant éventuellement encore convertis en un sel.

2. Procédé selon la revendication 1, caractérisé en ce qu'on prépare un composé de formule générale I dans laquelle :
X est -O- ;
R¹ est le groupe -CH₂-CO-R³ ;
R² est un hydrogène ou un radical alkyle en C₁-C₄, et
R³ est un radical alcoxy en C₁-C₄, hydroxy ou amino.

3. Procédé selon la revendication 1, caractérisé en ce qu'on prépare un composé de formule générale I dans laquelle :
X est -NH- ;
R¹ est le groupe -CH₂-CO-R³ ;
R² est un hydrogène et
R³ est un radical alcoxy en C₁-C₄ ou hydroxy.

4. Procédé selon la revendication 1, caractérisé en ce qu'on prépare l'acide thiényl-2 N-aminocarbonylméthylaminoacétique.

5. Procédé selon la revendication 1, caractérisé en ce qu'on prépare le chlorhydrate du thiényl-2 N-hydroxycarbonylméthylaminoacétamide.

6. Utilisation d'un dérivé de l'acide thiénylacétique préparé selon l'un des procédés selon les revendications 1 à 5, pour réaliser une préparation pharmaceutique destinée à lutter contre les maladies dues à une restriction de la fonction cérébrale, et à les prévenir, ou à lutter contre les processus de vieillissement cérébral.

7. Procédé pour réaliser une préparation pharmaceutique, caractérisé en ce qu'on transforme en une forme administrable un ou plusieurs dérivés de l'acide thiénylacétique de formule générale I, préparés par les procédés selon les revendications 1 à 5, ou leurs sels pharmaceutiquement acceptables, par mélange avec des excipients et adjuvants appropriés, pharmaceutiquement acceptables, et éventuellement encore avec une ou plusieurs autres substances à effet pharmaceutique.
